# EUROPEAN PATENT SPECIFICATION

(11) **EP 2 679 107 B1**
(45) Date of publication and mention of the grant of the patent: **17.06.2015**
(21) Application number: 12174367.8
(22) Date of filing: 29.06.2012
(51) Int. Cl.: A41D 13/12, A61B 5/0408, A41D 1/00, A61N 1/04

(54) **Electronic textile assembly**
Elektronische Textilanordnung
Ensemble textile électronique

(43) Date of publication of application: 01.01.2014
(73) Proprietor: Smart Solutions Technologies, S.L., 28290 Las Matas (Madrid) (ES)
(72) Inventor: Maciá Barber, Augustin, 28250 Torrelodones (ES); Llorca Juan, Daniel, 63822 Porto San Giorgio (IT); Vincente Rengel, Christian, 46200 Paiporta (ES)
(74) Representative: Schlich, George

(56) References cited:
- WO-A1-02/30279
- WO-A1-2004/110192
- WO-A1-2005/088772
- WO-A1-2009/020274
- WO-A1-2012/066056
- WO-A2-2007/050650
- US-A1- 2010 198 043
- ANJUM SALEEM ET AL: "Fabrication of Extrinsically Conductive Silicone Rubbers with High Elasticity and Analysis of Their Mechanical and Electrical Characteristics", POLYMERS, vol. 2, no. 3, 10 August 2010 (2010-08-10) , pages 200-210, XP55007217, DOI: 10.3390/polym2030200

## Description

The present invention relates to an electronic textile assembly.

### BACKGROUND ART

Currently, there are known in the state of the art wearable fabrics which comprise at least one sensor integrated into the fabric. The sensor allows to obtain physiological signals through their contact with the skin of the wearer, generally a human person, which are transported through electrically conductive tracks to an electrical connector which couples with an electronic instrument for receiving, collecting, storing, processing and/or transmitting data. Such data may be used to monitoring and/or evaluating the physiological signal of the user.

Using electrical connectors provided in the sensor, an electronic instrument may be removably connected to the garment. The electronic instrument may be used for receiving and/or processing and/or sending data from the sensors to a second electronic instrument. These second electronic instrument may be a mobile phone, PDA, personal computer or even a cloud-computing system.

Different sensors can be integrated into the wearable fabric, such as i.e. electrocardiogram sensor (ECG), electromyogram sensor (EMG), galvanic skin response sensor (GSR), electrochemical sensor, thermometer, skin impedance sensor, transpiration sensor, respiration sensor, a plurality of these, or yet other sensors.

Thus, there are known in the state of the art, examples of sensor apparatus which are incorporated in a garment, said apparatus comprising at least one sensor which in use of the garment are in contact with a user's skin, the sensor being in electrical contact with and electronic device through an electrically conductive track which comprises one or more electrical connectors to which the electronic device is attached.

A problem facing the developers of advanced electronic textiles is how to interconnect electrical components and electronic devices with each other and with electrical connectors via electrically conductive tracks provided on the fabric substrate of the electronic textile.

One of the drawbacks of the known systems where the track is made by a semi-conductive elastic material is the connector joint, since the connector is directly disposed on the track, i.e. the track is directly joined to the connector. This structure is mechanically weak when stretching the textile, and usually the textile is tore.

It is known in the flexible printed circuit (FPC) field, the weld area between track and other components must be reinforced with rigid elements increasing their tear resistance. Nevertheless, in the field of electronic textiles, when the substrate is a wearable, elastic and flexible textile garment, the integration of rigid elements is weak and most of the times the rigid element will break the textile when the garment is stretched.

The electronic devices are made of rigid plastics, rigid printed circuit boards (PCBs) and rigid connectors, so the device can support stress. This means that the union between a rigid electronic device and a wearable elastic sensor has to be improve.

Thus, from what is known in the art, it is derived that the development of an improved elastic semi-conductive track and electrical connector assembly in a wearable fabric is still of great interest.

WO 2009/020274 describes a garment for measuring physiological signals. The garment includes at least one electrode sensor, a signal connection line, a snap structure, and a measurement unit. The snap structure is coupled to a portion of the garment where the electrode sensor is not overlapped and is electrically connected to the signal line.

WO 2012/066056 describes a sensor for acquiring physiological signals using improved silicone rubber. The sensor may be included in a garment for wearing by a person involved in high levels of activity.

WO 02/30279 describes a health monitoring garment which employs a means of conducting electricity from the surface of the skin, through the fibres of a fabric to another fabric, which is removably attached to it and contains a microprocessor.

A paper in Polymers, vol. 2, no. 3, 10 August 2010 describes silicone rubbers reinforced with conductive fillers for use as sensors in textiles to detect the resistance change produced by stretching or relaxing. The variations of electrical resistance have been investigated by stretching and releasing the strands of conductive rubbers as a function of time. Two types of silicone rubbers-addition cured and condensation cured-were compounded with different electrically conductive fillers, among which carbon fibres have shown the best results. The carbon fibres improved the electrical conductance of the rubbers, even in very low weight percentages.

### SUMMARY OF THE INVENTION

In view of the above-mentioned and other drawbacks of the prior art, a general object of the present invention is to provide an improved elastic semi-conductive track and a flexible conductive support base assembly arranged on a textile fabric substrate, in particular reducing or substantially avoiding tearing the joining between the elastic track and rigid electrical components connected therein when the track, is streched, twisted, folded and/or squeezed while used.

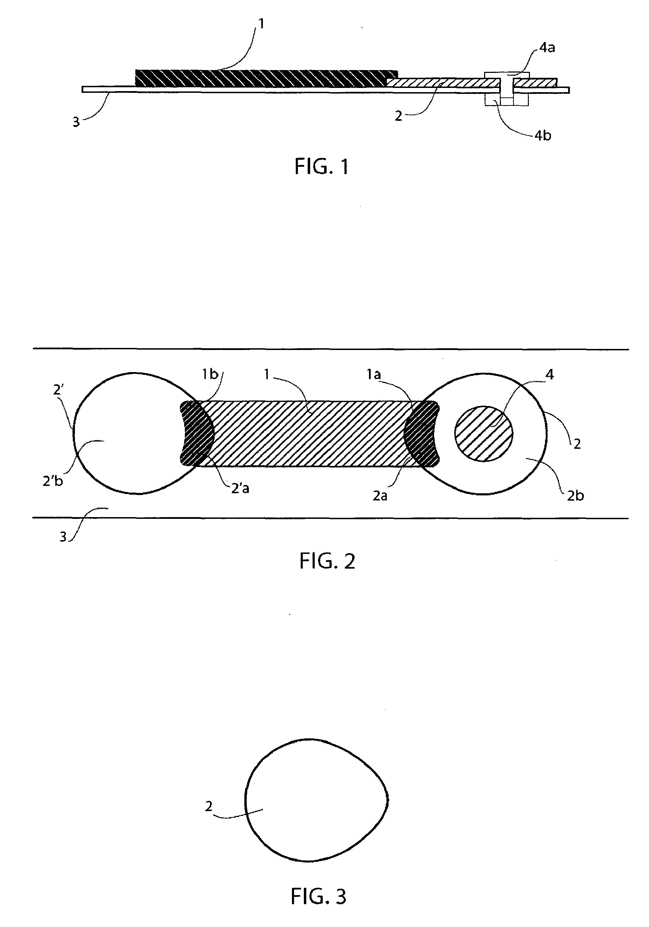

According to the first aspect of the present invention there is provided an elastic semi-conductive track (1) and flexible conductive support base (2) assembly arranged on a textile fabric substrate (3), the flexible conductive support base (2) being a textile comprising conductive and non-conductive fibres and having at least one of its ends round shaped (2a), wherein at least one end (1 a) of the track (1) is treading on said at least one round shaped end (2a) of at least one flexible conductive support base (2), and a non-treaded area (2b) by the track of the at least one flexible conductive support base (2) is in electrical contact with a rigid electrical component (4). See Figs. 1 and 2.

According to a second aspect of the invention, it is provided a physiological signal sensor adapted to be incorporated in a garment, wherein said sensor comprises the assembly as defined above, wherein the electrode is adapted to obtain physiological signals through its contact with the skin (6) of the wearer of the garment.

In another aspect, it is provided a device comprising the physiological signal sensor as defined herein, and an electronic instrument for receiving, collecting, storing, processing and/or transmitting data from said sensor.

The present invention provides the device as defined above adapted to be integrated in a garment so as to be placed in contact with the skin of a user during the use of the garment. Therefore, another aspect of the invention relates to a garment comprising at least the device defined above.

Furthermore, another aspect of the present invention provides a method for monitoring a physiological signal of a user comprising
a) receiving, collecting, storing, processing and/or transmitting one or more parameters indicative of at least one physiological signal of a user originating from at least one sensor as defined in the present invention incorporated in a garment; and
b) evaluating said physiological signal along the time.

### BRIEF DESCRIPTION OF THE DRAWINGS

FIG. 1 illustrates a cross-section of an embodiment of the track (1) and support base (2) assembly arranged on a textile fabric substrate (3), wherein the support base is in electrical contact with a rigid electrical component comprising two parts (4a and 4b).
FIG. 2 illustrates an elevation view of the assembly of the invention according to an embodiment wherein both ends of the track (1 a and 1 b) are treading on two different support bases (2 and 2'), and a rigid electrical component (4) is arranged on the non-treaded area (2b) of one of the support bases.
FIG. 3 illustrates the teardrop-like shape of the support base according to an embodiment of the invention.
FIG. 4 illustrates an elevation view of the garment according to an embodiment of the present invention.
FIG. 5 illustrates a cross-section of an embodiment of a sensor according to the present invention.

### DETAILED DESCRIPTION OF THE INVENTION

In accordance with the present invention, the flexible conductive support base (2) comprises two areas, one being treaded by the elastic semi-conductive track (2a) and the other one (2b) either being adapted to connect a rigid electrical component (4) or being adapted to be used as electrode (5).

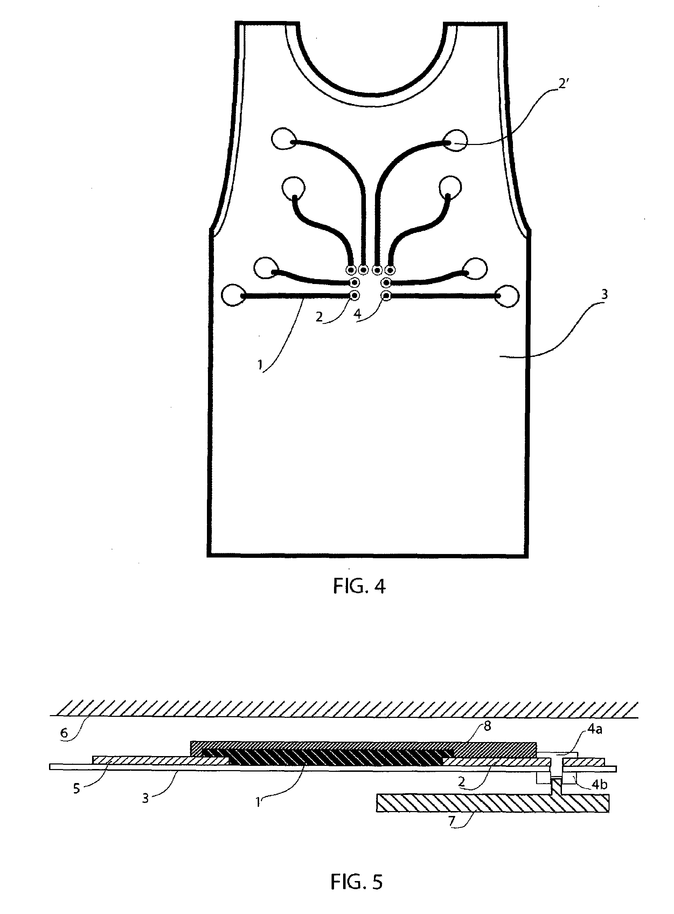

Thus, according to an embodiment of the present invention, the elastic semi-conductive track and flexible conductive support base assembly of the present invention is that wherein each end of the track (1 a and 1 b) are treading on two different flexible conductive support bases (2 and 2'). See Figs. 2 and 5.

In a preferred embodiment of this realization, the non-treaded area (2'b) of one of the flexible conductive support bases (2'), is adapted to be used as electrode (2'b = 5) and on the non-treaded area (2b) of the other flexible conductive support base (2) there is arranged a rigid electrical component (4). See Figs. 2 and 5.

In another embodiment of the present invention, the elastic semi-conductive track and flexible conductive support base assembly of the present invention is that wherein one end of the track (1 a) is treading on the at least one end round shaped (2a) of one flexible conductive support base (2), whereas on the non-treaded area (2b) of such support base there is arranged a rigid electrical component (4); and the other end of the track (1 b) is adapted to be in electrical contact with an electrode (5).

The expert in the art knows several rigid electrical components (4), which can be arranged in electrical contact with the assembly of the invention, nonlimiting examples of such components are electrical connectors, switches, resistors, capacitors, passive components (protection devices), magnetic (inductive) devices, piezoelectric devices, crystals, resonators, power sources, semiconductors (diodes, transistors, integrated circuits, optoelectronic devices), display devices, antennas, transducers, sensors, electrochemical sensors, detectors and electrodes.

As defined above, the conductive support base (2) is a flexible and conductive textile comprising conductive and non-conductive fibres, having at least one of its ends (2a), the end which is treaded by the track, round shaped, being preferably a teardrop-like shape (see Fig. 3). On the other hand, according to some embodiments of the present invention, shape and dimension of the track may vary and it is not limited by the manufacturing process of the fabric substrate.

In accordance with a particular embodiment of the present invention, the conductive support base has a teardrop-like shape, wherein the connection edging between support base and track has round shape. Resulting in an improvement of the mechanical resistance to stretching, minimizing or substantially avoiding tearing the joints when the track is streched, twisted, folded and/or squeezed while used. Furthermore, the circuit design is simplifyed since the support base can be guided to the track direction and viceversa.

According to an embodiment of the present invention, the conductive support base is attached to the textile fabric substrate with a textile adhesive. According to a particular embodiment, the textile adhesive is any suitable hot-melt adhesive for use in textile known in the art.

The term "fabric substrate" should, in the context of the present invention, be understood as a material or product manufactured by textile fibres. The fabric substrate may, for example, be manufactured by means of weaving, braiding, knitting or any other known method in the art.

The skilled person in the art knows several fabrics adequate for the invention, preferably elastic fabric. Thus, in some embodiments of the invention, the elastic fabric is for example LYCRA® and/or polyamide, and/or polyester and/or nylon. In other embodiment, other textiles may be used. In some other embodiments of the invention, the elastic fabric is a fabric, which comprises a percentage of elastane, preferably comprised of from 3% w/w to 20% w/w.

As mentioned above, the conductive support base (2) is a flexible and conductive textile comprising conductive and non-conductive fibres.

Non limiting examples of conductive fibers are fibre made of silver, copper, nickel, stainless steel, gold, non conductive fibres coated with a conductive material or mixtures thereof. Non limiting examples of coating conductive materials are silver, cooper, nickel, stainless stell, gold and silicone rubber loaded with carbon or silver powder. Particularly preferred conductive fibres are those which comprise silver coated nylon.

Non limiting examples of non conductive fibres are wool, silk, cotton, flax, jute, acrylic fibre, polyamide polyester, nylon and/or elastic yarns (such as LYCRA® branded spandex from Invista™ S.a.r.l).

According to a preferred embodiment of the present invention, the conductive fibres comprise silver coated nylon, such as X-static ® yarns from Laird Sauquoit Industries, and the non-conductive fibres comprise nylon.

Therefore, according to a preferred embodiment, the track is elastic and flexible. The elasticity and flexibility of the electrically conductive track allow that conductivity is not interrupted with the movement of the fabric. The track may be provided to the fabric in any manner known in the art, preferably is applied to the surface of the fabric substrate through screen-printing methods.

Placing the rigid electrical component (4), preferably an electrical connector, on the conductive support base (2), which is electrically in contact with the elastic and semi-conductive track (1) instead of directly in contact with the track, results in an improvement of the mechanical properties of the assembly, avoiding the textile being tore when stretching.

Thus, the conductive support base is used as a conductive foot print which is in electrical contact with the elastic and electrically conductive track, being the conductive support base wherein the rigid electrical component (4) is arranged on.

If the flexible conductive support base is elastic the assembly will work perfectly on its own but when we place a rigid electrical component, such as an electrical connector, the stress will move from the joint between track and support base to the joint between the support base and the rigid electrical component, the mechanical properties of a joint between an elastic and a rigid element is low when the assembly suffered mechanical stress. Being this assembly integrated into a textile the mechanical properties of the joints between materials are crucial to obtain a proper electrical circuit. For all this reasons the best case is to reduce from elastic to flexible and from flexible to rigid, this transaction is done by the flexible and non-elastic conducive support base.

In a preferred embodiment, the elastic and electrically semi-conductive track is made of a silicone rubber loaded with a conductive material, preferably a room temperature curing silicone loaded with carbon fibres, carbon black, nickel coated graphite, copper fibres and mixtures thereof. In a more preferred embodiment, the conductive silicone is a silicone rubber loaded with carbon black, such as VP97065/30 from Alpina Technische Produkte GmbH.

The amount of conductive material in the silicone rubber is not important once you reach the percolation threshold; this value depends on the conductive filler and the silicone rubber material. In a particular embodiment of the present invention, the silicone rubber is loaded with an amount comprised of from 5% w/w to 40% w/w of an electrically conductive material.

Preferably, the thickness of the elastic and electrically conductive track layer is comprised of from 50 to 800 µm thick, more preferably comprised of from 100 to 500 µm thick, from 120 to 400 µm thick, from 150 to 300 µm thick, being particularly preferred comprised of from 120 to 180 µm thick.

In accordance with a particular embodiment, other alternatives known in the art such as conductive inks can be used as track, although their elasticity is worst than that of the silicone.

Thus, in accordance with an embodiment of the present invention, the assembly is that wherein a silicone rubber loaded with an electrically conductive material, preferably selected from those above mentioned, is screen-printed on the textile fabric substrate and treading on the at least one round shaped end of the conductive support base.

The silicone in the liquid state when is printing in the fabric is capable to penetrate in the orifices of the fabric, anchoring with the structure of the conductive layer. Therefore, according to a particular embodiment of the present invention, the track is integrated into the textile fabric substrate and partially into the at least one round shaped end of the conductive support base by anchoring the silicone with the structure of the fibres of the textile fabric substrate and the conductive support base when cured the silicone at room temperature after being screen-printed on them.

Thus, the silicone rubber loaded with the conductive material is screen-printing on the fabric substrate (3) and treading partially on the one round shaped end (2a) of the conductive support base (2); thus, the silicone penetrates in the orifices of the textiles, and anchors with the structure of the fibres of the textiles when curing at room temperature after being screen-printed on them. Preferably, the flexible semi-conductive track (1) is provided to the surface of the fabric substrate (3) and the at least one round shaped end (2a) of the conductive support base (2) by screen-printing a silicone rubber loaded with an electrically conductive material, comprising a step of applying pressure when applying the silicone rubber directly to the textile fabric substrate and the at least one round shaped end of the conductive support base, in order to eliminate any air bubble that will break the conductivity. For this purpose the preferred application system is a screen-printing process using low speed and high pressure. The pressure to be applied is comprised of from 0.2 to 0.8 Kg/m², preferably comprised of from 0.3 to 0.5 Kg/m²; being particularly preferred a pressure of about 0.45 Kg/m².

In the context of the present invention, a textile is to be understood to comprise any kind of woven, knitted, or tufted cloth, or a non-woven fabric (e.g. a cloth made of fibres that have been bonded into a fabric). Textile in this context furthermore also comprises yarns, threads and wools that can be spun, woven, tufted, tied and otherwise used to manufacture cloth. Also in the present context, "elastic material" is to be understood as a material which relatively easily may be stretched or compressed and is able to resume its original shape.

Using a textile made of conductive and non-conductive fibres as the textile conductive support base (2), the attachment of the elastic and electrically conductive track made of a silicone loaded with a conductive material is mechanically steady, due to the anchoring of the silicone with the structure of the fibres of the textile.

The term "electrical connector" as used herein, refers to an electro-mechanical device, which provides a separable interface between two electronic subsystems, sensor and electronic instrument, without an unacceptable effect on signal integrity.

In some embodiments, said electrical connectors are electrically conductive fasteners. Preferably, said electrically conductive fasteners are press studs (also sometimes referred to as snaps, snap fasteners, or poppers). Such press studs are commonly made of a pair of interlocking discs. A circular lip under one disc (4a) fits into a groove on the top of the other (4b), holding them fast until an amount of force is applied (see Figs. 1 and 5). The press-studs may be attached to fabric by hammering, plying, or sewing. In other embodiments, other kinds of fasteners may be used, such as e.g. magnets, a pin-socket or plug-socket connection (e.g. with the socket being provided on the sensor apparatus), conductive Velcro® or other conductive metal clip fasteners. Preferably, any kind of fastener that allows an electronic device to be easily attached and detached may be used. In some embodiments, in use said electronic device is attached on the outside of the garment. In these embodiments, the electronic device may be particularly easily attached and detached by a user, even possibly during exercise.

As mentioned above, in another aspect of the invention it is provided a physiological signal sensor adapted to be incorporated in a garment, the sensor comprising the assembly as defined above which comprises an electrode (5), either the non-treaded area (2'b) of one of the two flexible conductive support bases (2') when each end of the track (1 a and 1 b) are treaded in two different support bases (2 and 2') or an electrode in electrical contact with the second end of the track (1 b) when only one support base is present; the electrode (5) being adapted to obtain physiological signals through its contact with the skin (6) of the wearer of the garment, preferably a human person. See Fig. 5.

In a preferred embodiment, the physiological signal sensor is that wherein the track (1) is electrically isolated from its contact with the skin (6) of the wearer of the garment, and the rigid electrical component (4) is an electrical connector adapted to transmit a physiological signal obtained through the electrode (5) to an electronic instrument (7). In a preferred embodiment the track is covered with an insulating material (8), preferably an isolating silicone rubber (see Fig. 5).

In some embodiments of the invention, the flexible conductive support base (2) is attached to a textile fabric substrate (3) with a textile adhesive, preferably a holt-melt adhesive.

The term "hot-melt adhesive" as used herein, refers to a thermoplastic, non-structural adhesive that flows when heated and hardens and strengthens as it cools.

A device comprising at least one sensor as defined above and an electronic instrument for receiving, collecting, storing, processing and/or transmitting data from said sensor, is also herein provided. The present invention also relates to a garment comprising the device substantially as hereinbefore described. In preferred embodiments, said device is arranged in said garment such that in use the device is arranged substantially in an area which may be suitable locations for the measuring of various parameters, preferably a user's electrocardiogram (ECG).

Some preferred garments are: a t-shirt with long sleeves, a t-shirt with short sleeves, a tank top, a leotard, a bra, a sleeveless top, a halter top, a spaghetti-strapped shirt, a singlet, an A-shirt or a tube top.

In the acquisition of some physiological signals, such as the ECG, the resistance of both the electrode and track is one of the elements to determine the noise of the signals. In accordance with an embodiment, the resistance of the electrode is comprised from 0.5 Ω to 15 Ω, preferably comprised from 1 Ω to 10 Ω. In another preferred embodiment the resistance of the track is comprised from 1 Ω to 50 kΩ, preferably comprised from 5 Ω to 10 kΩ.

As mentioned above, according to some embodiments of the present invention, the track is provided to the surface of the fabric substrate through screen-printing methods.

The term "screen-printing", as commonly known in the art, refers to a process made using a stencil in which image or design is print on a very fine mesh screen and the printable material is squeezed onto the printing surface through the area of the screen that is not covered by the stencil.

In accordance with a particular embodiment of the present invention, the method of preparation of the track and electrical connector assembly comprises:
a) die cut at least one conductive support base;
b) fix the at least one conductive support base to a fabric support with a textile adhesive, preferably a holt melt adhesive applying pressure and heating from 80 to 185 Celsius degree, preferably 110-165 Celsius degree, for 5-30 seconds, preferably 10-20 seconds;
c) screen-printing a conductive silicone rubber on the textile fabric substrate while partially is treading in the at least one round shaped end of the conductive support base, applying a pressure comprised of from 0.2 to 0.8 Kg/m².

In a preferred embodiment, the process further comprises a step of curing the conductive silicone rubber at room temperature. In the present invention the silicone rubber is cured into de fabric. When it is required to decrease the time of cured, a step of pre-cured by heating at a temperature comprised of from 80°C to 200°C is included. Preferably, the pre-cured step is carried out at a temperature comprised of from 90°C to 165°C.

Using the device and garment of the invention, the physiological signals detected can be at least one of the following: cardiac pulse, respiratory frequency, electrodermal response (EDR), measures electrical skin conductivity, electrocardiography (ECG), electromyography (EMG). These signals refer to electrical signals produced in the body. Preferably the physiological signal is an ECG signal.

The electronic instrument is adapted to receive, collect, process, storage, and/or transmit data from sensors incorporated in garment. For example, an ECG signal will be received. The skilled person knows different storage, processing, and/or transmitting means, which can be incorporated in the electronic instrument.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

Example 1. Comparative test between an elastic semi-conductive track directly in contact with a rigid electrical connector (assembly 1) and the elastic semi-conductive track and flexible conductive support base assembly of the invention wherein the rigid electrical connector is in contact with the support base (assembly 2).

The assemblies where prepared with elastic semi-conductive track made with the conductive silicone rubber loaded with carbon black, VP97065/30 from Alpina Technische Produkte GmbH; the assembly 2 included a flexible support base which was prepared with a conductive textile made with the conductive fibres of silver coated nylon commercialized as X-static® by Laird Sauquoit Industries, and non-conductive fibres of nylon; whereas the substrate in both assemblies was made with polyester, nylon and LYCRA® fibres.

The tracks were 80 mm long and 15 mm wide. Tests were repeated 3 times.

Resistivity between both extremes of the track was measured in order to evaluate the durability of the assembly. Resistivity increases with elongations of material, in case of a break the resistivity is drastically increased. Generally, resistency values should not exced 25 kΩ.

Each test consists of applying three different stretching cycles of different stretching. The first cycle of 30 repetitions subjecting the specimens to 140% elongation. Results are in Table 1.

**Table 1.**

| **Assembly 1** | **100%** | **140%** |
|---|---|---|
| 001 | 1.7 kΩ | 7 kΩ |
| 002 | 2.2 kΩ | 4.7 kΩ |
| 003 | 1.6 kΩ | 5.8 kΩ |
| | | |

| **Assembly 2** | **100%** | **140%** |
|---|---|---|
| 001 | 1.5 kΩ | 2.3 kΩ |
| 002 | 1 kΩ | 1.6 kΩ |
| 003 | 1.5 kΩ | 2.3 kΩ |

The cycle of 30 repetitions subjecting the specimens to 200% elongation. Results are in Table 2.

**Table 2.**

| **Assembly 1** | **100%** | **200%** |
|---|---|---|
| 001 | 1.7 kΩ | 13.8 kΩ |
| 002 | 2.2 kΩ | 18.2 kΩ |
| 003 | 1.6 kΩ | 10.4 kΩ |
| | | |

| **Assembly 2** | **100%** | **200%** |
|---|---|---|
| 001 | 1.5 kΩ | 6.1 kΩ |
| 002 | 1 kΩ | 4.2 kΩ |
| 003 | 1.5 kΩ | 5.9 kΩ |

The third cycle of 5 repetitions subjecting the specimens to 250% elongation. Results are in Table 3.

**Table 3.**

| **Assembly 1** | **100%** | **250%** |
|---|---|---|
| 001 | 1.7 kΩ | 33.2 kΩ |
| 002 | 2.2 kΩ | 930 MΩ (break) |
| 003 | 1.6 kΩ | 29.4 kΩ |
| | | |

| **Assembly 2** | **100%** | **250%** |
|---|---|---|
| 001 | 1.5 kΩ | 10.6 kΩ |
| 002 | 1 kΩ | 8.3 kΩ |
| 003 | 1.5 kΩ | 10.1 kΩ |

Results show that at 250% elongations of assembly 2 allows the system to be below the 25 kΩ while the assembly 1 in two of the three samples exceeded the limit of 25 kΩ and in one case the rupture occurred in the track zone with the electrical connector.

## Claims

1. Elastic semi-conductive track (1) and flexible conductive support base (2) assembly arranged on a textile fabric substrate (3), the flexible conductive support base (2) being a textile comprising conductive and non-conductive fibres and having at least one of its ends round shaped (2a), wherein at least one end (1 a) of the track (1) is treading on said at least one round shaped end (2a) of at least one flexible conductive support base (2), and a non-treaded area (2b) by the track of the at least one flexible conductive support base (2) is in electrical contact with a rigid electrical component (4).

2. The assembly according to claim 1, wherein each end of the track (1a and 1b) are treading on two different flexible conductive support bases (2 and 2').

3. The assembly according to claim 2, wherein on the non-treaded area (2b) of one of the flexible conductive support bases (2) there is arranged a rigid electrical component (4), and the non-treaded area (2'b) of the other flexible conductive support base (2') is adapted to be used as an electrode (5).

4. The assembly according to claim 1, wherein one end (1a) of the track (1) is treading on the at least one round shaped end (2a) of one flexible conductive support base (2) wherein it is arranged as a rigid electrical component (4), and the other end (1b) of the track (1) is adapted to be in electrical contact with an electrode (5).

5. The assembly according to any one of claims 1-4, wherein the flexible conductive support base (2) is attached to the textile fabric substrate (3) with a textile adhesive.

6. The assembly according to any one of claims 1-5, wherein the track (1) comprises a layer of silicone rubber loaded with an electrically conductive material.

7. The assembly according to any one of claims 1-6, wherein the track (1) comprises a layer of a room temperature curing silicone loaded with an electrically conductive material selected from carbon fibres, carbon black, nickel coated graphite, copper fibres and mixtures thereof.

8. The assembly according to any one of claims 6-7, wherein the track (1) is integrated into the textile fabric substrate (3) and partially into the at least one round shaped end (2a) of the flexible conductive support base (2) by anchoring the silicone with the structure of the fibres of the textile fabric substrate (3) and the flexible conductive support base (3) when cured the silicone at room temperature after being screen-printed on them.

9. The assembly according to claim 8, wherein the silicone is screen-printed on the textile fabric substrate (3) and on the at least one round shaped end (1a) of the flexible conductive support base (2) applying a pressure comprised of from 0.2 to 0.8 Kg/m2.

10. A physiological signal sensor adapted to be incorporated in a garment, said sensor comprising the assembly as defined in any of the claims 3-9, wherein the electrode (5) is adapted to obtain physiological signals through its contact with the skin (6) of the wearer of the garment.

11. The physiological signal sensor according to claim 10, wherein the track (1) is electrically isolated from its contact with the skin (6) of the wearer of the garment, and the rigid electrical component (4) is an electrical connector adapted to transmit a physiological signal obtained through the electrode (5) to an electronic instrument (7).

12. A device comprising the sensor as defined in any of claims 10-11, and an electronic instrument (7) for receiving, collecting, storing, processing and/or transmitting data from said sensor.

13. A garment comprising the device of claim 12.

14. A method for monitoring a physiological signal of a user comprising
- receiving, collecting, storing, processing and/or transmitting one or more parameters indicative of at least one physiological signal of a user originating from at least one sensor as defined in any of the claims 10-11 incorporated in a garment; and
- evaluating said physiological signal along the time.

15. The method according to claim 14 wherein the physiological signal is an ECG signal.

## Patentansprüche

1. Anordnung einer elastischen halbleitenden Bahn (1) und einer biegsamen leitfähigen Stützbasis (2), die auf einem Textilgewebesubstrat (3) angeordnet ist, wobei die biegsame leitfähige Stützbasis (2) ein Textil ist, das leitfähige und nicht leitende Fasern umfasst und mindestens ein Ende aufweist, das rund geformt (2a) ist, wobei mindestens ein Ende (1a) der Bahn (1) auf dem mindestens einen rund geformten Ende (2a) mindestens einer biegsamen leitfähigen Stützbasis (2) ein Profil bildet und ein profilloser Bereich (2b) durch die Bahn der mindestens einen biegsamen leitfähigen Stützbasis (2) steht in elektrischem Kontakt mit einem starren elektrischen Bauteil (4).

2. Anordnung nach Anspruch 1, wobei jedes Ende der Bahn (1a und 1b) auf zwei verschiedenen biegsamen leitfähigen Stützbasen (2 und 2') ein Profil bildet.

3. Anordnung nach Anspruch 2, wobei auf dem profillosen Bereich (2b) einer der biegsamen leitfähigen Stützbasen (2) ein starres elektrisches Bauteil (4) angeordnet ist und wobei der profillose Bereich (2'b) der anderen biegsamen leitfähigen Stützbasis (2') geeignet ist, um als eine Elektrode (5) verwendet zu werden.

4. Anordnung nach Anspruch 1, wobei ein Ende (1a) der Bahn (1) auf dem mindestens einen rund geformten Ende (2a) einer biegsamen leitfähigen Stützbasis (2) ein Profil bildet, wobei es als ein starres elektrisches Bauteil (4) angeordnet ist und das andere Ende (1b) der Bahn (1) ist geeignet, um mit einer Elektrode (5) in elektrischem Kontakt zu stehen.

5. Anordnung nach einem beliebigen der Ansprüche 1-4, wobei die biegsame leitfähige Stützbasis (2) an dem Textilgewebesubstrat (3) mit einem Textilklebstoff befestigt ist.

6. Anordnung nach einem beliebigen der Ansprüche 1-5, wobei die Bahn (1) eine Schicht aus Silikonkautschuk umfasst, die mit einem elektrisch leitfähigen Material versehen ist.

7. Anordnung nach einem beliebigen der Ansprüche 1-6, wobei die Bahn (1) eine Schicht aus einem bei Raumtemperatur aushärtendem Silikon umfasst, die mit einem elektrisch leitfähigen Material versehen ist, das aus Kohlenstofffasern, Industrieruß, mit Nickel beschichtetem Graphit, Kupferfasern und Gemischen davon ausgewählt wird.

8. Anordnung nach einem beliebigen der Ansprüche 6-7, wobei die Bahn (1) in dem Textilgewebesubstrat (3) und teilweise in das mindestens eine rund geformte Ende (2a) der biegsamen leitfähigen Stützbasis (2) integriert ist, indem das Silikon mit der Struktur der Fasern des Textilgewebesubstrats (3) und der biegsamen leitfähigen Stützbasis (3) verankert wird, wenn das Silikon bei Raumtemperatur ausgehärtet ist, nachdem es auf diese mit Siebdruck aufgetragen wird.

9. Anordnung nach Anspruch 8, wobei das Silikon mit Siebdruck auf das Textilgewebesubstrat (3) und das mindestens eine rund geformte Ende (1a) der biegsamen leitfähigen Stützbasis (2) unter Anwendung eines Drucks, der 0,2 bis 0,8 kg/m² entspricht, aufgetragen wird.

10. Physiologischer Signalsensor, der geeignet ist, um in einem Kleidungsstück integriert zu werden, wobei der Sensor die Anordnung nach einem beliebigen der Ansprüche 3-9 umfasst, wobei die Elektrode (5) geeignet ist, um durch ihren Kontakt mit der Haut (6) des Trägers des Kleidungsstücks physiologische Signale zu erhalten.

11. Physiologischer Signalsensor nach Anspruch 10, wobei die Bahn (1) elektrisch von ihrem Kontakt mit der Haut (6) des Trägers des Kleidungsstücks isoliert ist und das starre elektrische Bauteil (4) ist ein elektrisches Verbindungselement, das geeignet ist, um ein physiologisches Signal, dass durch die Elektrode (5) erhalten wird, an ein elektronisches Instrument (7) zu übertragen.

12. Vorrichtung, welche den Sensor nach einem beliebigen der Ansprüche 10-11 umfasst und elektronisches Instrument (7) für das Empfangen, Sammeln, Speichern, Verarbeiten und/oder Übertragen von Daten von dem Sensor.

13. Kleidungsstück, das die Vorrichtung nach Anspruch 12 umfasst.

14. Verfahren zur überwachung eines physiologischen Signals eines Verwenders, das Folgendes umfasst
- Empfangen, Sammeln, Speichern, Verarbeiten und/oder Übertragen eines oder mehrerer Parameter, die mindestens ein physiologisches Signal eines Verwenders anzeigen, das von mindestens einem Sensor nach einem beliebigen der Ansprüche 10-11 ausgegeben wurde, der in einem Kleidungsstück integriert ist;
und
- Auswerten des physiologischen Signals mit der Zeit.

15. Verfahren nach Anspruch 14, wobei das physiologische Signal ein EKG-Signal ist.

## Revendications

1. Un ensemble avec piste semi-conductrice élastique (1) et support conducteur flexible (2) disposé sur un substrat en étoffe (3), le support conducteur flexible (2) étant un textile comprenant des fibres conductrices et non conductrices, et dont au moins un des bouts est de forme arrondie (2a), au moins une extrémité (1a) de la piste (1) foulant au moins un bout arrondi (2a) d'au moins un support conducteur flexible (2), et une zone (2b) non foulée par la piste d'au moins un support conducteur flexible (2) est en contact électrique avec un composant électrique rigide.

2. L'ensemble selon la revendication 1, chaque bout de la piste (1a et 1 b) foulant deux supports conducteurs flexibles divers (2 et 2').

3. L'ensemble selon la revendication 2, dans lequel sur la zone non foulée (2b) d'un des supports conducteurs flexibles (2) un composant électrique rigide (4) est disposé, et la zone non foulée (2'b) de l'autre support conducteur flexible (2') est adaptée pour être utilisée comme électrode (5).

4. L'ensemble selon la revendication 1, un bout (1a) de la piste (1) foulant au moins un bout arrondi (2a) d'un support conducteur flexible (2), où il est disposé comme un composant électrique rigide (4), et l'autre bout (1b) de la piste (1) est adapté pour être en contact électrique avec un électrode (5).

5. L'ensemble selon une quelconque des revendications 1 à 4, dans lequel le support conducteur flexible (2) est fixé sur le substrat en étoffe (3) avec un adhésif textile.

6. L'ensemble selon une quelconque des revendications 1 à 5, dans lequel la piste (1) comprend une couche de caoutchouc silicone renforcé avec un matériau conducteur.

7. L'ensemble selon une quelconque des revendications 1 à 6, dans lequel la piste (1) comprend une couche de silicone durcissable à température ambiante renforcée avec un matériau conducteur sélectionné parmi les suivants : fibres de carbone, noir de carbone, graphite recouvert au nickel, fibres de cuivre et des mélanges de ces derniers.

8. L'ensemble selon une quelconque des revendications 6-7, dans lequel la piste (1) est intégrée dans le substrat d'étoffe textile (3) et partiellement dans au moins un bout arrondi (2a) du support conducteur flexible (2), en ancrant le silicone à la structure des fibres du substrat d'étoffe textile (3) et le support conducteur flexible (3) lors du durcissement du silicone à la température ambiante après l'impression au tamis sur ceux-ci.

9. L'ensemble selon la revendication 8, dans lequel le silicone est imprimé au tamis sur le substrat d'étoffe textile (3), au moins un bout arrondi (1a) du support conducteur flexible (2) appliquant une pression comprise entre 0,2 et 0,8 kg/m².

10. Un capteur de signaux physiologiques adapté pour être incorporé dans un vêtement, ledit capteur comprenant l'ensemble défini selon une quelconque des revendications 3 à 9, dans lequel l'électrode (5) est adaptée pour obtenir des signaux physiologiques à travers son contact avec la peau (6) de la personne portant le vêtement.

11. Le capteur de signaux physiologiques selon la revendication 10, dans lequel la piste (1) est électriquement isolée de son contact avec la peau (6) de la personne portant le vêtement, et le composant électrique rigide (4) est un connecteur électrique adapté pour transmettre un signal physiologique, obtenu par le biais de l'électrode (5), à un instrument électronique (7).

12. Un dispositif comprenant le capteur selon la définition des revendications 10-11, et un instrument électronique (7) permettant de recevoir, saisir, stocker, traiter et/ou transmettre des données provenant dudit capteur.

13. Un vêtement comprenant le dispositif selon la revendication 12.

14. Une méthode pour le contrôle d'un signal physiologique d'un utilisateur, comprenant
La réception, la saisie, le stockage, le traitement et/ou la transmission d'un ou plusieurs paramètres indicatif d'au moins un signal physiologique d'un utilisateur, provenant d'au moins un capteur, selon une quelconque des revendications 10-11, incorporé dans un vêtement, et
L'évaluation dudit signal physiologique le long du temps.

15. La méthode selon la revendication 14, le signal physiologique étant un signal d'ECG.
